# EUROPEAN PATENT SPECIFICATION

(11) **EP 0 591 581 B1**
(45) Date of publication and mention of the grant of the patent: **09.08.1995**
(21) Application number: 92309150.8
(22) Date of filing: 08.10.1992
(51) Int. Cl.: C07C 217/48, A61K 31/135

(54) **N-Alkyl-3-phenyl-3-(2-alkylthiophenoxy)propylamines**
N-Alkyl-3-Phenyl-3-(1-Alkylthiophenoxy)propylamine
N-Alkyl-3-Phényl-3-(1-Alkylthiophénoxy)propylamine

(43) Date of publication of application: 13.04.1994
(73) Proprietor: ELI LILLY AND COMPANY, Indianapolis, Indiana 46285 (US)
(72) Inventor: Gehlert, Donald Richard, Indianapolis, Indiana 46202 (US); Robertson, David Wayne, Poway, California 92064 (US); Wong, David Taiwai, Indianapolis, Indiana 46226 (US)
(74) Representative: Tapping, Kenneth George

(56) References cited:
- US-A- 4 194 009
- US-A- 4 194 009

## Description

The invention relates to novel N-alkyl-3-phenyl-3-(2-substituted phenoxy)propylamines which are selective and potent inhibitors of norepinephrine uptake.

In the past few decades, understanding of the biological role of nerve cells (neurons) has greatly increased. Specifically, particular neurons have been implicated in particular diseases. The present invention provides for compounds which inhibit presynaptic biogenic amine uptake in at least one type of neuron, the norepinephrine neuron.

Norepinephrine neurons are found everywhere in the brain, and are also known to exist in other organs of the body, such as the bladder. The compounds of the present invention indirectly stimulate the neurons by inhibiting norepinephrine uptake. Moreover, the adrenal glands are known to secrete norepinephrine in response to stress. Thus, norepinephrine is also called noradrenalin.

Patients with Alzheimer's and Korsakoff's syndrome and depression may have deficiencies of norepinephrine. The present invention is useful in treatment of disorders associated with norepinephrine imbalance. Schildkraut, *Neuropharmacology of Affective Disorders*, 427 (1973) is an excellent source of background information.

US-A-4,018,895 describes and claims a class of N-alkyl-3-phenyl-3-phenoxypropylamines similar to those claimed in the present invention. In particular, Example 2 discloses N-methyl-3-phenyl-3-(2-methoxyphenoxy)propylamine.

According to the present invention there is provided a compound of the formula (I) 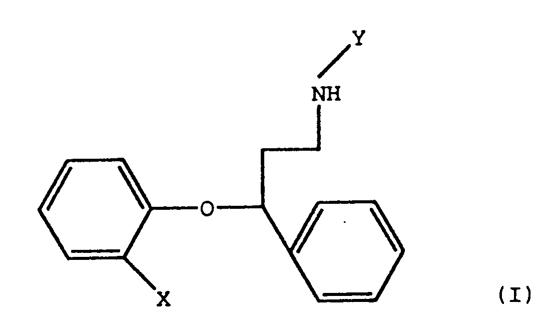
wherein X is C₁-C₄ alkylthio, and Y is C₁-C₂ alkyl or a pharmaceutically acceptable acid addition salt thereof.

An advantage of these compounds is that they are more potent norepinephrine uptake inhibitors than the methoxy-substituted cogeners.

The invention also provides pharmaceutical formulations comprising a compound of the formula (I), or a pharmaceutically acceptable salt thereof, and a pharmaceutically acceptable carrier, diluent or excipient therefor. Further embodiments of the invention are methods for selectively inhibiting the uptake of norepinephrine as well as for treating a variety of disorders which have been linked to decreased neurotransmission of norepinephrine in mammals including substance abuse, depression, narcolepsy, panic disorder, bulimia, and related psychiatric disorders. The compounds of the present invention are useful in treating these mental diseases. In addition, because of the known interaction of the norepinephrine with the urinary system, the compounds are useful to treat urinary incontinence.

Preferred compounds are those wherein Y is methyl. The most preferred compound of this series is N-methyl-3-phenyl-3-(2-methylthiophenoxy)propylamine.

The compounds of this invention can exist as the individual stereoisomers as well as the racemic mixture. Accordingly, the compounds of the present invention will include not only the d,l-racemates, but also their respective optically active d- and l-isomers.

As pointed out above, the invention includes the pharmaceutically acceptable acid addition salts of the compounds defined by the above formula. Since the compounds of this invention are amines, they are basic in nature and accordingly react with any number of inorganic and organic acids to form pharmaceutically acceptable acid addition salts. Since the free amines of the invention are typically oils at room temperature, it is preferable to convert the free amines to their corresponding pharmaceutically acceptable acid addition salts, which are routinely solid at room temperature, for ease of handling. Acids commonly employed to form such salts include inorganic acids such as hydrochloric, hydrobromic, hydroiodic, sulfuric and phosphoric acid, as well as organic acids such as paratoluenesulfonic, methanesulfonic, oxalic, parabromophenylsulfonic, carbonic, succinic, citric, benzoic and acetic acid, and related inorganic and organic acids.

Such pharmaceutically acceptable salts thus include sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, terephthathalate, sulfonate, xylenesulfonate, phenylacetate, phenylpropionate, phenylbutyrate, citrate, lactate, β-hydroxybutyrate, glycollate, maleate, tartrate, methanesulfonate, propanesulfonates, naphthalene-1-sulfonate, naphthalene-2-sulfonate, mandelate and the like salts. Preferred pharmaceutically acceptable acid addition salts include those formed with mineral acids such as hydrochloric acid and hydrobromic acid, and especially those formed with organic acids such oxalic acid and maleic acid.

The following compounds further illustrate compounds contemplated within the scope of the present invention:
N-ethyl-3-phenyl-3-(2-methylthiophenoxy)propylamine phosphate, N-methyl-3-phenyl-3-(2-t-butylthiophenoxy)propylamine hydrochloride, N-ethyl-3-phenyl-3-(2-n-propylthiophenoxy)propylamine formate, N-methyl-3-phenyl-3-(2-ethylthiophenoxy)propylamine succinate, N-methyl-3-phenyl-3-(2-isopropylthiophenoxy)propylamine hydrochloride.

The compounds of this invention in the form of their free bases are high boiling oils, but white crystalline solids in the form of their acid addition salts. The compounds can be prepared in several ways. One useful procedure for preparing compounds represented by the above formula is substantially carried out as described in U.S. Patent Serial No. 4,018,895.

According to a further embodiment of the present invention there is provided a process for preparing a compound of formula (I) which comprises reacting a 3-phenylpropylamine derivative of the formula: 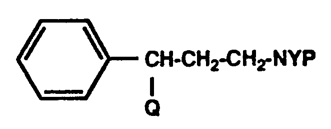
with a compound of the formula: 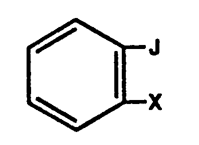
where Q and J are hydroxy or halo; and P is hydrogen or a protecting group; followed in the case where P is a protecting group by deprotection, and in the case where P is halo by amination with an amine of formula YNH₂, and optionally, where it is desired to form a pharmaceutically - acceptable acid addition salt, by salification of any free base present.

### EXAMPLE 1

### Preparation of N-methyl-3-phenyl-3-(2-methylthiophenoxy)propylamine hydrochloride

A 10.80 g portion of chloropropiophenone was dissolved in 100 ml of methanol in a 500 ml, 3 necked, round-bottomed flask. The flask was fitted with a nitrogen inlet and a thermometer. The reaction mixture was stirred via magnetic stirring rod, and was held under an atmosphere of nitrogen. While the mixture was cooled in an ice bath, 2.03 g of sodium borohydride was added very slowly. The mixture was removed from the ice bath and then stirred for approximately two hours at room temperature.

The mixture was then evaporated to a yellow oil and diluted with approximately 100 ml of water. The mixture was extracted from the water by washing three times with ether. The ether was then washed two times with water and one time with saturated sodium chloride solution. The resulting mixture was dried over sodium sulfate and evaporated to 11.2 g of yellow intermediate product comprising 3-chloro-1-phenyl-1-propanol.

A 4.99 g portion of the intermediate 3-chloro-1-phenyl-1-propanol as prepared above was placed into a 3-necked, 250 ml round-bottomed flask which had been flushed with nitrogen. The flask was fitted with a thermometer, a nitrogen inlet and an addition funnel. The compound, 3.52 g of 2-methylthiophenol and 7.69 g of triphenylphosphine were magnetically stirred in 40 ml of tetrahydrofuran. A 4.61 ml portion of diethylazodicarboxylate was added dropwise to this mixture. The temperature of the reaction mixture was kept around 25°C using an ice bath. The addition funnel was rinsed with tetrahydrofuran and the reaction mixture was stirred overnight at approximately room temperature. During the addition of diethylazodicarboxylate, the mixture became thick, opaque and yellow in color. After approximately one hour, the mixture cleared to a yellow solution. The reaction mixture was evaporated to a yellow solid. Hexane was added to the solid and the mixture was shaken vigorously.

The insoluble triphenylphosphine oxide was then suction-filtered, hexane was again added to the solid, and the mixture was shaken and re-filtered. The filtrates were evaporated to 6.63 g of clear oil. The clear oil was then dissolved in ether. A 2 N sodium hydroxide solution was then added. The sodium hydroxide layer was removed and the organic layer was washed once with water and once with saturated sodium chloride solution. The solution was then dried over sodium sulfate. The resulting compound, 1-(3-chloro-1-phenylpropoxy)-2-methylthiobenzene, was then aminated by reacting with methylamine (40% in water) in ethanol at 130°C for for 3 hours. After evaporating the ethanol, water was added to the resulting yellow oil. The mixture was extracted two times with ether, and washed two times with water and once with brine solution. The product was dried over Sodium sulfate.

The product was purified via flash chromatography by wet loading using a methylene chloride, methanol and ammonium hydroxide system with the ratio being 100:5:1. A 870 mg portion of product was recovered.

The amine was dissolved in methanol and 1.05 equivalents 12 N HCl were added. Upon recrystallization, an 850 mg portion of off-white crystals was obtained.

The melting point of this solid was 143° to 144.5°C.

| Analysis: | | | |
|---|---|---|---|
| Theory: | C, 63.04; | H, 6.85; | N, 4.32; |
| Found: | C, 63.08; | H, 6.94; | N, 4.23. |

As noted above, the compounds of this invention are useful for inhibiting the uptake of norepinephrine. Therefore, another embodiment of the present invention is a method for inhibiting norepinephrine uptake in mammals which comprises administering to a mammal requiring increased neurotransmission of norepinephrine a pharmaceutically effective amount of a compound of the invention.

The term "pharmaceutically effective amount", as used herein, represents an amount of a compound of the invention which is capable of inhibiting norepinephrine uptake. The particular dose of compound administered will, of course, be determined by the particular circumstances surrounding the case, including the compound administered, the route of administration, the particular condition being treated, and similar considerations. The compounds can be administered by a variety of routes, including the oral, rectal, transdermal, subcutaneous, intravenous, intramuscular or intranasal routes. The oral route of administration is preferred.

The compounds of the invention inhibit the uptake of norepinephrine in mammals in an unexpectedly selective and potent manner. A typical daily dose will contain from about 0.01 mg/kg to about 20 mg/kg of the active compound of this invention. Preferred daily doses will be from about 0.05 mg/kg to 10 mg/kg, ideally from about 0.1 mg/kg to 5 mg/kg.

A variety of physiological functions have been shown to be influenced by norepinephrine levels in the body. As such, the compounds of the present invention are believed to have the ability to treat a variety of disorders in mammals associated with abnormal norepinephrine levels in the body.

The following experiment was conducted to demonstrate the ability of the compounds of the present invention to inhibit the uptake of norepinephrine. This general procedure is set forth by Wong *et al*., 6 *Drug Development Research* 397 (1985).

Male Sprague-Dawley rats weighing 150-250 g were decapitated and brains were immediately removed. Cerebral cortices were homogenized in 9 volumes of a medium containing 0.32 M sucrose and 10 mM glucose. Crude synaptosomal preparations were isolated after differential centrifugation at 1000 X g for 10 minutes and 17,000 X g for 28 minutes. The final pellets were suspended in the same medium and kept in ice until use within the same day.

Synaptosomal uptake of ³H-norepinephrine (³H-NE) was determined as follows. Cortical synaptosomes (equivalent to 1 mg of protein) were incubated at 37°C for 5 minutes in 1 ml of Krebs-bicarbonate medium containing also 10 mM glucose, 0.1 mM iproniazide, 1 mM ascorbic acid, 0.17 mM EDTA and 50 nM ³H-NE. The reaction mixture was immediately diluted with 2 ml of ice-chilled Krebs-bicarbonate buffer and filtered under vacuum with a cell harvester (Brandel, Gaithersburg, MD). Filters were rinsed twice with approximately 5 ml of ice-chilled 0.9% saline and the uptake of the radio-labeled NE assessed by liquid scintillation counting. Accumulation of ³H-NE at 4°C was considered to be background and was subtracted from all measurements. The concentration of the test compound required to inhibit 50% of the ³H-NE accumulation (IC₅₀ value) was determined by linear regression analysis.

The results of the evaluation are set forth below in Table I. As shown in the Table, the compound was evaluated to determine concentration of the test compound needed to inhibit 50% of norepinephrine, as indicated by IC₅₀.

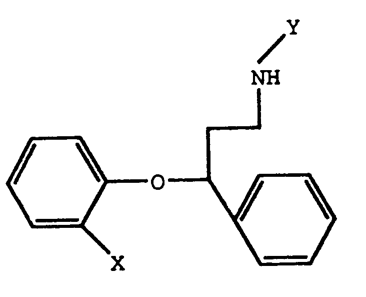

| Compound | | IC₅₀ NE Uptake (nM) |
|---|---|---|
| X | Y | |
| CH₃S | CH₃ | 4.4 |
| CH₃O | CH₃ | 7.0 |

These data clearly show that the methylthio derivatives of the invention are significantly more active than the methoxy derivative of the art.

The compounds of the present invention are preferably formulated prior to administration. Therefore, yet another embodiment of the present invention is a pharmaceutical formulation comprising a compound of the invention and a pharmaceutically acceptable carrier, diluent or excipient therefor.

The present pharmaceutical formulations are prepared by known procedures using well known and readily available ingredients. In making the compositions of the present invention, the active ingredient will usually be mixed with a carrier which may be in the form of a capsule, sachet, paper or other container. When the carrier serves as a diluent, it may be a solid, semisolid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, ointments containing, for example up to 10% by weight of the active compound, soft and hard gelatin capsules, suppositories, sterile injectable solutions and sterile packaged powders.

Some examples of suitable carriers, excipients, and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, methyl cellulose, methyl- and propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide quick, sustained or delayed release of the active ingredient after administration to the patient by employing procedures well known in the art.

The compositions are preferably formulated in a unit dosage form, each dosage containing from about 5 to about 500 mg, more usually from about 25 to about 300 mg, of the active ingredient. The term "unit dosage form" refers to physically discrete units suitable as unitary dosages for human subjects and other mammals, each unit containing a predetermined quantity of active material calculated to produce the desired therapeutic effect, in association with a suitable pharmaceutical carrier.

The following formulation examples are illustrative only and are not intended to limit the scope of the invention in any way.

### Formulation 1

Hard gelatin capsules are prepared using the following ingredients:

| | Quantity (mg/capsule) |
|---|---|
| N-methyl-3-phenyl-3-(2-methylthiophenoxy)propylamine | 250 |
| starch, dried | 200 |
| magnesium stearate | 10 |
| Total | 4̅6̅0̅ mg |

The above ingredients are mixed and filled into hard gelatin capsules in 460 mg quantities.

### Formulation 2

Tablets each containing 60 mg of active ingredient are made as follows:

| | |
|---|---|
| N-methyl-3-phenyl-3-(2-methylthiophenoxy)propylamine | 60.0 mg |
| starch | 45.0 mg |
| microcrystalline cellulose | 35.0 mg |
| polyvinylpyrrolidone (as 10% solution in water) | 4.0 mg |
| sodium carboxymethyl starch | 4.5 mg |
| magnesium stearate | 0.5 mg |
| talc | 1.0 mg |
| Total | 150.0 mg |

The active ingredient, starch and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50°C and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

## Claims

1. A compound of formula (I) 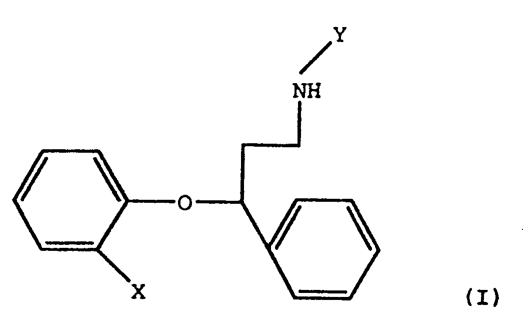 wherein X is C₁-C₄ alkylthio and Y is C₁-C₂ alkyl or a pharmaceutically acceptable acid addition salt thereof.

2. N-methyl-3-phenyl-3-(2-methylthiophenoxy)propylamine, or a pharmaceutically acceptable acid addition salt thereof.

3. A compound as claimed in claim 1 or 2 for use as an norepinephrine uptake inhibitor.

4. A compound as claimed in claim 1 or 2 for use in the treatment for depression, panic disorder, narcolepsy, substance addiction, urinary incontinence, or bulimia.

5. A pharmaceutical formulation comprising a compound as claimed in claim 1 or 2 or a pharmaceutically acceptable acid addition salt thereof associated with one or more pharmaceutically acceptable carriers, diluents or excipients therefor.

6. A process for preparing a compound of formula (I) as claimed in claim 1 or 2, which comprises reacting a 3-phenylpropylamine derivative of the formula: 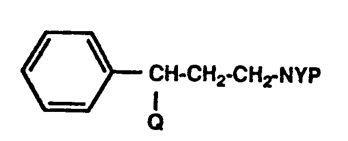 with a compound of the formula: 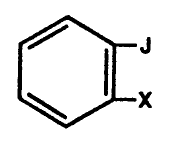 where Q and J are hydroxy or halo; X and Y are as defined in claim 1 or 2; and P is hydrogen, halo or a protecting group; followed in the case where P is a protecting group by deprotection, and in the case where P is halo by amination and optionally, where it is desired to form a pharmaceutically-acceptable acid addition salt, by salification of any free base present.

## Patentansprüche

1. Verbindung der Formel (I) 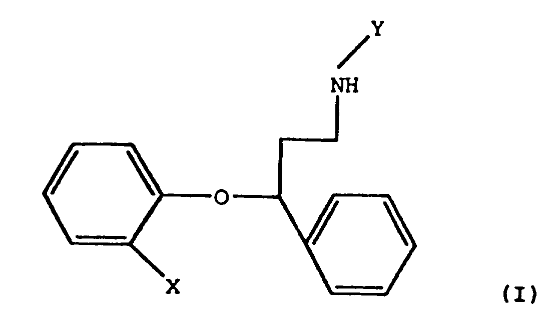 worin X für C₁-C₄-Alkylthio steht und Y für C₁-C₂-Alkyl steht, oder ein pharmazeutisch annehmbares Säureadditionssalz hiervon.

2. N-Methyl-3-phenyl-3-(2-methylthiophenoxy)propylamin oder ein pharmazeutisch annehmbares Säureadditionssalz hiervon.

3. Verbindung nach Anspruch 1 oder 2 zur Verwendung als Inhibitor für eine Aufnahme von Norepinephrin.

4. Verbindung nach Anspruch 1 oder 2 zur Verwendung bei der Behandlung von Depression, Angstzuständen, Narkolepsie, Abhängigkeit von Substanzen, Harninkontinenz oder Bulimie.

5. Pharmazeutische Formulierung, enthaltend eine Verbindung nach Anspruch 1 oder 2 oder ein pharmazeutisch annehmbares Säureadditionssalz hiervon, in Kombination mit einem oder mehreren pharmazeutisch annehmbaren Trägern, Verdünnungsmitteln oder Hilfsstoffen hierfür.

6. Verfahren zur Herstellung einer Verbindung der Formel (I) nach Anspruch 1 oder 2, dadurch gekennzeichnet, daß man ein 3-Phenylpropylaminderivat der Formel 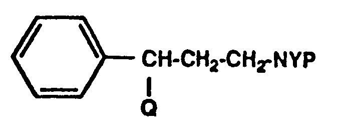 mit einer Verbindung der Formel 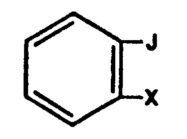 worin Q und J Hydroxy oder Halogen sind, X und Y die in den Ansprüchen 1 und 2 angegebenen Bedeutungen haben und P Wasserstoff, Halogen oder eine Schutzgruppe ist, umsetzt und, falls P eine Schutzgruppe ist, diese Gruppe abspaltet und, falls P für Halogen steht, diese Verbindung aminiert und wahlweise, falls man ein pharmazeutisch annehmbares Säureadditionssalz haben möchte, eine vorhandene freie Base einer Salzbildung unterzieht.

## Revendications

1. Composé répondant à la formule (I) 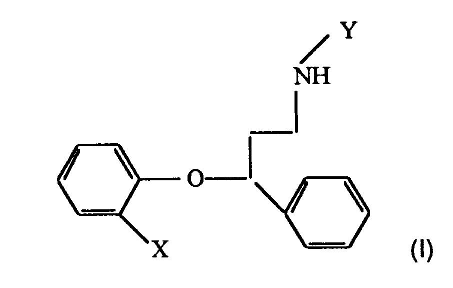 dans laquelle X est un C₁-C₄ alkylthio et Y est un C₁-C₂ alkyle, ou un sel d'addition acide pharmaceutiquement acceptable de celui-ci.

2. N-méthyl-3-phényl-3-(2-méthylthiophénoxy)propylamine, ou un sel d'addition acide pharmaceutiquement acceptable de celui-ci.

3. Composé tel que revendiqué à la revendication 1 ou 2 destiné à l'utilisation en tant qu'inhibiteur d'absorption de norépinéphrine.

4. Composé tel que revendiqué à la revendication 1 ou 2 destiné à l'utilisation dans le traitement de la dépression, du trouble de l'affolement, de la narcolepsie, de la dépendance aux substances, de l'incontinence urinaire ou de la boulimie.

5. Formulation pharmaceutique qui comprend un composé tel que revendiqué à la revendication 1 ou 2, ou un sel d'addition acide pharmaceutiquement acceptable de celui-ci, associé à un ou plusieurs supports, diluants ou excipients, pharmaceutiquement acceptables pour celui-ci.

6. Procédé pour la préparation d'un composé répondant à la formule (I) tel que revendiqué à la revendication 1 ou 2, qui comprend la réaction d'un dérivé de la 3-phénylpropylamine de formule : 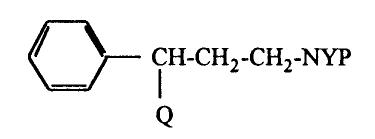 avec un composé répondant à la formule : 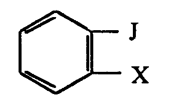 où Q et J sont un hydroxy ou un halogène, X et Y sont tels que définis à la revendication 1 ou 2; et P est un hydrogène, un halogène ou un groupe protecteur; cette réaction est suivie d'une déprotection dans le cas où P est un groupe protecteur, et d'une amination dans le cas où P est un halogène, et facultativement d'une salification par toute base libre présente au cas où l'on désire former un sel d'addition acide pharmaceutiquement acceptable.
